# FASCICULE DE BREVET EUROPEEN

(11) **EP 1 115 375 B1**
(45) Date de publication et mention de la délivrance du brevet: **15.03.2006**
(21) Numéro de dépôt: 99943007.7
(22) Date de dépôt: 20.09.1999
(51) Int. Cl.: A61Q 19/00, A61K 8/365, A61K 8/37

(54) **UTILISATION D'AU MOINS UN DERIVE DE L'ACIDE 10-HYDROXY-2-DECENOIQUE DANS UNE COMPOSITION DESTINEE A FAVORISER LA DESQUAMATION DE LA PEAU**
VERWENDUNG VON MINDESTENS EINEM DERIVAT DER 10-HYDROXY-2-DECENOICSÄURE IN EINER ZUSAMMENSETZUNG ZUR FÖRDERUNG DER ABSCHUPPUNG DER HAUT
USE OF AT LEAST A10-HYDROXY-2- DECENOIC ACID DERIVATIVE IN A COMPOSITION FOR PROMOTING SKIN SCALING

(30) Priorité: 22.09.1998 FR 9811810
(43) Date de publication de la demande: 18.07.2001
(73) Titulaire: L'OREAL, 75008 Paris (FR)
(72) Inventeur: BRETON, Lionel, 78000 Versailles (FR); PINEAU, Nathalie, 86000 Poitiers (FR); BENECHIE, Emile, 91190 Gif (FR); LI, Martine, F-92350 Le Plessis Robinson (FR); PICOT, Françoise, 78460 Chevreuse (FR); POTIER, Pierre, 75007 Paris (FR)
(74) Mandataire: Warcoin, Jacques
(86) Numéro de dépôt international: PCT/FR1999/002230
(87) Numéro de publication internationale: WO 2000/016739

(56) Documents cités:
- FR-A- 1 401 515
- FR-A- 2 073 240
- DATABASE CHEMICAL ABSTRACTS [Online] STN, Karlsruhe, DE abrégé 129: 85 846, XP002125395 & JP 10 147514 A (POLA CHEMICAL IND., INC.) 2 juin 1998 (1998-06-02)
- S. HOWE ET AL: "Composition of freshly harvested and commercial royal jelly" JOURNAL OF APICULTURE RESEARCH, vol. 24, no. 1, 1985, pages 52-61, XP000863079
- DATABASE CHEMICAL ABSTRACTS [Online] STN abrégé 106: 55 626, XP002107315 & JP 61 176510 A (POLA CHEM. IND., INC.) 8 août 1986 (1986-08-08)
- DATABASE CHEMICAL ABSTRACTS [Online] STN abrégé 111: 83 876, XP002107316 & CN 87 100 420 A (QINGYUN MA) 13 janvier 1988 (1988-01-13)
- DATABASE CHEMICAL ABSTRACTS [Online] STN abrégé 68: 89829, XP002107317 & CS 123 434 A (V. KANTNER) 15 juin 1967 (1967-06-15)

## Description

L'invention concerne l'utilisation dans une composition, d'une quantité efficace d'au moins un dérivé de l'acide 10-hydroxy-2-décénoique, le dérivé ou la composition étant destinés à favoriser la desquamation de la peau et/ou stimuler le renouvellement épidermique et/ou lutter contre le vieillissement de la peau. Elle se rapporte aussi à des compositions pour favoriser la desquamation de la peau et/ou stimuler le renouvellement épidermique et donc lutter contre le vieillissement cutané intrinsèque et/ou extrinsèque, ainsi qu'à un procédé de traitement non thérapeutique de la peau destiné à favoriser la desquamation et/ou lutter contre le vieillissement de la peau.

La desquamation est un phénomène naturel lié au fait que l'épiderme, qui constitue la couche supérieure de la peau, est en constante régénération. L'épiderme est constitué de plusieurs assises de cellules, dont la plus profonde est l'assise basale constituée de cellules indifférenciées. Au cours du temps, ces cellules vont différencier et migrer vers la surface de l'épiderme en constituant les différentes assises de celui-ci, jusqu'à former à la surface de l'épiderme les cornéocytes qui sont des cellules mortes qui s'éliminent par desquamation. Cette perte en surface est compensée par la migration de cellules de l'assise basale vers la surface de l'épiderme. Il s'agit du renouvellement perpétuel de la peau. Une élimination forcée de la couche cornée accélère le renouvellement et permet de lutter contre le vieillissement.
Dans le même temps ces cellules poursuivent leur différenciation dont le dernier stade est le cornéocyte. Il s'agit en fait de cellules mortes qui constituent la dernière couche de l'épiderme, c'est à dire la couche la plus externe encore appelée *stratum corneum.*

Le vieillissement cutané résultant de facteurs intrinsèques ou extrinsèques se traduit par l'apparition de rides et ridules, par le jaunissement de la peau qui développe un aspect parcheminé accompagné de l'apparition de taches pigmentaires, par la désorganisation des fibres d'élastine et de collagène entraînant une perte d'élasticité, de souplesse et de fermeté ou par l'apparition de télangiectasies.

Certains de ces signes du vieillissement sont plus particulièrement liés au vieillissement intrinsèque ou physiologique, c'est-à-dire au vieillissement « normal » lié à l'âge ou chronobiologique, alors que d'autres sont plus spécifiques du vieillissement extrinsèque, c'est-à-dire du vieillissement provoqué d'une manière générale par l'environnement ; il s'agit plus particulièrement du photovieillissement dû à l'exposition au soleil, à la lumière ou à tout autre rayonnement.

L'invention s'intéresse au vieillissement intrinsèque ou physiologique ainsi qu'au vieillissement extrinsèque.

Les changements de la peau dus au vieillissement intrinsèque sont la conséquence d'une sénescence génétiquement programmée où interviennent des facteurs endogènes. Ce vieillissement intrinsèque provoque notamment un ralentissement du renouvellement des cellules de la peau, ce qui se traduit essentiellement par l'apparition d'altérations cliniques telles que la réduction du tissu adipeux sous-cutané et l'apparition de fines rides ou ridules, et par des changements histopathologiques tels qu'une augmentation du nombre et de l'épaisseur des fibres élastiques, une perte de fibres verticales de la membrane du tissu élastique, et la présence de grands fibroblastes irréguliers dans les cellules de ce tissu élastique.

Au contraire, le vieillissement extrinsèque entraîne des altérations cliniques telles que des rides épaisses et la formation d'une peau molle et tannée, et des changements histopathologiques tels qu'une excessive accumulation de matière élastique dans le derme supérieur et une dégénérescence des fibres de collagène.

On connaît dans l'art antérieur divers agents destinés à lutter contre le vieillissement cutané.

Ainsi, le brevet US-A-4603146 décrit l'emploi d'acide rétinoïque et de ses dérivés dans des compositions cosmétiques, en vue de lutter contre le vieillissement cutané.
Par ailleurs, de nombreux brevets et publications (voir par exemple la demande EP-A-413528) ainsi que de nombreuses compositions cosmétiques du commerce enseignent l'emploi des α-hydroxyacides comme l'acide lactique, l'acide glycolique ou encore l'acide citrique pour traiter le vieillissement cutané.

On connaît enfin les β-hydroxyacides et plus spécialement l'acide salicylique ainsi que ses dérivés pour leur propriétés desquamantes (voir les documents WO-A-93/10756 et US-A-4 767 750).

Tous ces composés ont une action contre le vieillissement de la peau en favorisant la desquamation, c'est-à-dire l'élimination des cellules « mortes » situées à la surface de la couche cornée de l'épiderme. Cette propriété "desquamante" est aussi appelée, souvent à tort, propriété kératolytique.

Mais les composés de l'art antérieur présentent également des effets secondaires, qui consistent en des picotements, des tiraillements, des échauffements et des rougeurs désagréables pour l'utilisateur.

On constate donc que subsiste le besoin d'agents antivieillissement ayant une action au moins aussi efficace que celle des composés de l'art antérieur, mais ne présentant pas leurs inconvénients.

L'invention a pour but de pallier les inconvénients des solutions de l'art antérieur et de proposer une composition favorisant la desquamation de la peau et/ou stimulant le renouvellement épidermique, dont l'utilisation n'entraînerait pas de picotements, de tiraillements, d'échauffements ou de rougeurs désagréables pour l'utilisateur.

La demanderesse a trouvé de manière inattendue que l'application d'une quantité efficace de dérivés particuliers de l'acide 10-hydroxy-2-décénoique permet de favoriser la desquamation de la peau et/ou stimuler le renouvellement épidermique donc de lutter contre le vieillissement.

Dans les publications de l'art antérieur les dérivés de l'acide 10-hydroxy-2-décénoique sont connus pour leur propriété comme activateur du système immunitaire.

A la connaissance de la demanderesse, il n'a jamais été décrit dans l'art antérieur l'utilisation de dérivés de l'acide 10-hydroxy-2-décénoique pour favoriser la desquamation de la peau et/ou stimuler le renouvellement épidermique et donc lutter contre le vieillissement cutané intrinsèque et/ou extrinsèque.

L'invention a donc pour premier objet l'utilisation dans une composition, d'une quantité efficace de dérivés de l'acide 10-hydroxy-2-décénoique de formule décrites ci-dessous, le dérivé ou la composition étant destinés à favoriser la desquamation de la peau et/ou stimuler le renouvellement épidermique et donc lutter contre le vieillissement cutané intrinsèque et/ou extrinsèque.

L'invention concerne donc les composés de formules suivantes :
10-hydroxy-dec-2-énoate de 2-diméthylaminoéthyle de formule : 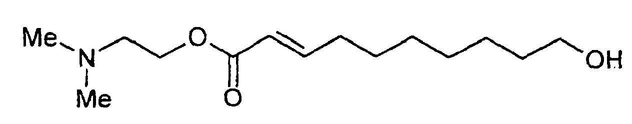
10-hydroxy-dec-2-ènoate de 2,3-dihydroxypropyle de formule : 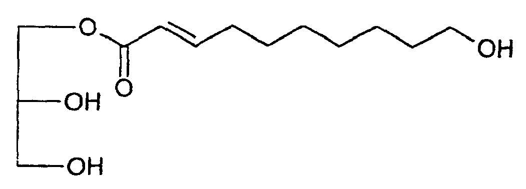
1,3-di-(10-hydroxy-dec-2-ènoate) de 2-hydroxypropyle de formule : 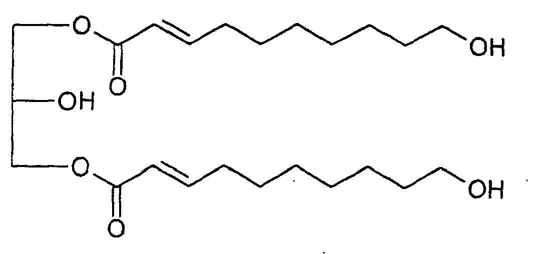
1,2,3-tri-(10-hydroxy-dec-2-ènoate) de propyle de formule : 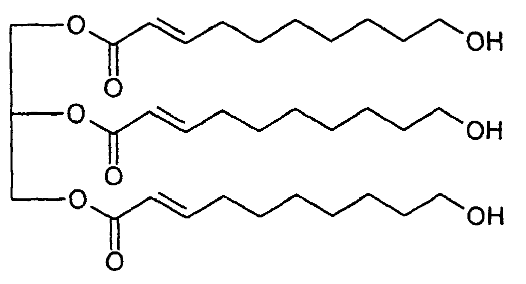
1,2-di-(10-hydroxy-dec-2-ènoate) de 3-[(2-méthoxy)-éthoxy-méthoxy]-propyle de formule :

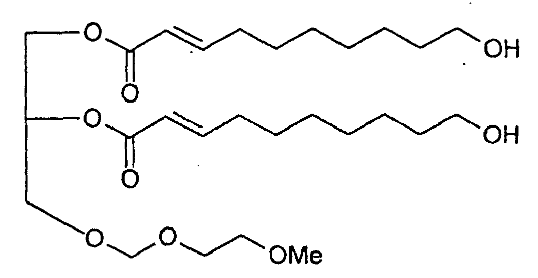

Par la suite dans le texte, le terme dérivés de l'acide 10-hydroxy-2-décénoique s'entend comme désignant les composés ci-dessus décrits, d'origine naturelle ou synthétique, purifiés ou toute préparation les contenant.
Par origine naturelle, on entend un composé extrait de matériel naturel dans lequel il se trouve présent. Par origine synthétique oh entend un composé préparé par synthèse chimique ou par biotechnologie.

Bien entendu, il est possible d'utiliser selon l'invention les dérivés de l'acide 10-hydroxy-2-décénoique seuls ou en mélange.

De même, les dérivés de l'acide 10-hydroxy-2-décénoique peuvent être utilisés sous leur forme Cis et/ou Trans.

La quantité de dérivés de l'acide 10-hydroxy-2-décénoique utilisable selon l'invention, dépend bien évidemment de l'effet recherché et doit être en une quantité efficace pour favoriser la desquamation de la peau et/ou stimuler le renouvellement épidermique et donc lutter contre le vieillissement cutané intrinsèque et/ou extrinsèque.

A titre d'exemple la quantité de dérivés de l'acide 10-hydroxy-2-décénoique utilisable selon l'invention peut aller par exemple de 0,001% à 10% et de préférence de 0,01 % à 1 % du poids total de la composition.

Selon un autre aspect, l'invention à pour objet une composition pour favoriser la desquamation de la peau et/ou stimuler le renouvellement épidermique et donc lutter contre le vieillissement cutané intrinsèque et/ou extrinsèque, comprenant au moins un dérivé de l'acide 10-hydroxy-2-décénoique.

Dans la composition selon l'invention le dérivé de l'acide 10-hydroxy-2-décénoique peut être présent en une quantité de 0,001 % à 10% et de préférence de 0,01% à 1% du poids total de la composition.

Selon encore un autre aspect, l'invention a pour objet un procédé de traitement non thérapeutique pour favoriser la desquamation de la peau et/ou stimuler le renouvellement épidermique et donc lutter contre le vieillissement cutané intrinsèque et/ou extrinsèque, caractérisé en ce qu'on applique sur la peau une composition cosmétique comprenant au moins un dérivé de l'acide 10-hydroxy-2-décénoique.

Ladite composition peut se présenter sous toutes les formes galéniques connues comme par exemple sous la forme d'une émulsion, notamment huile-dans-eau ou eau-dans-huile, voire sous la forme d'une émulsion multiple.
Elle peut également se présenter sous forme d'une solution aqueuse, éventuellement gélifiée, ou sous forme d'une lotion, par exemple biphasée, d'une crème, d'un lait, voire d'une mousse.

Les compositions de l'invention peuvent être ingérées, injectées ou appliquées sur la peau (sur toute zone cutanée du corps), les cheveux, les ongles ou les muqueuses (buccale, jugale, gingivale, génitale, conjonctive). Selon le mode d'administration, les compositions selon l'invention peuvent se présenter sous toutes les formes galéniques normalement utilisées.

Pour une application topique sur la peau, la composition peut avoir la forme notamment de solution aqueuse ou huileuse ou de dispersion du type lotion ou sérum, d'émulsions de consistance liquide ou semi-liquide du type lait, obtenues par dispersion d'une phase grasse dans une phase aqueuse (H/E) ou inversement (E/H), ou de suspensions ou émulsions de consistance molle du type crème ou gel aqueux ou anhydres, ou encore de microcapsules ou microparticules, ou de dispersions vésiculaires de type ionique et/ou non ionique. Ces compositions sont préparées selon les méthodes usuelles.

Elles peuvent être également utilisées pour les cheveux sous forme de solutions aqueuses, alcooliques ou hydroalcooliques, ou sous forme de crèmes, de gels, d'émulsions, de mousses ou encore sous forme de compositions pour aérosol comprenant également un agent propulseur sous pression.
La composition selon l'invention peut aussi être une composition pour soins capillaires, et notamment un shampooing, une lotion de mise en plis, une lotion traitante, une crème ou un gel coiffant, une composition de teintures (notamment teintures d'oxydation) éventuellement sous forme de shampooings colorants, des lotions restructurantes pour les cheveux, une composition de permanente (notamment une composition pour le premier temps d'une permanente), une lotion ou un gel antichute, un shampooing antiparasitaire, etc.

Pour l'injection, la composition peut se présenter sous forme de lotion aqueuse, huileuse ou sous forme de sérum. Pour les yeux, elle peut se présenter sous forme de gouttes et pour l'ingestion, elle peut se présenter sous forme de capsules, de granulés de sirops ou de comprimés.

Les quantités des différents constituants des compositions selon l'invention sont celles classiquement utilisées dans les domaines considérés.

Les compositions selon l'invention peuvent également consister en des préparations solides constituant des savons ou des pains de nettoyage.

Les compositions peuvent aussi être conditionnées sous forme d'une composition pour aérosol comprenant également un agent propulseur sous pression.

Lorsque la composition est une émulsion, la proportion de la phase grasse peut aller de 5% à 80% en poids, et de préférence de 5% à 50% en poids par rapport au poids total de la composition. Les huiles, les cires, les émulsionnants et les coémulsionnants utilisés dans la composition sous forme d'émulsion sont choisis parmi ceux classiquement utilisés dans le domaine cosmétique. L'émulsionnant et le coémulsionnant sont présents, dans la composition, en une proportion allant de 0,3% à 30% en poids, et de préférence de 0,5 à 20% en poids par rapport au poids total de la composition. L'émulsion peut, en outre, contenir des vésicules lipidiques.

Lorsque la composition est une solution ou un gel huileux, la phase grasse peut représenter plus de 90% du poids total de la composition.

Les compositions de l'invention peuvent être à usage cosmétique ou pharmaceutique. Préférentiellement, les compositions de l'invention sont des compositions à usage cosmétique.

De façon connue, la composition cosmétique peut contenir également des adjuvants habituels dans le domaine cosmétique, tels que les gélifiants hydrophiles ou lipophiles, les additifs hydrophiles ou lipophiles, les conservateurs, les antioxydants, les solvants, les parfums, les charges, les filtres, les absorbeurs d'odeur et les matières colorantes. Les quantités de ces différents adjuvants sont celles classiquement utilisées dans le domaine cosmétique, et par exemple de 0,01 % à 10% du poids total de la composition.' Ces adjuvants, selon leur nature, peuvent être introduits dans la phase grasse, dans la phase aqueuse et/ou dans les sphérules lipidiques.
Comme huiles ou cires utilisables dans l'invention, on peut citer les huiles minérales (huile de vaseline), les huiles végétales (fraction liquide du beurre de karité, huile de tournesol), les huiles animales (perhydrosqualène), les huiles de synthèse (huile de Purcellin), les huiles ou cires siliconées (cyclométhicone) et les huiles fluorées (perfluoropolyéthers), les cires d'abeille, de carnauba ou paraffine. On peut ajouter à ces huiles des alcools gras et des acides gras (acide stéarique). Comme émulsionnants utilisables dans l'invention, on peut citer par exemple le stéarate de glycérol, le polysorbate 60 et le mélange de PEG-6/PEG-32/Glycol Stéarate vendu sous la dénomination de Tefose^{R} 63 par la société Gattefosse.

Comme solvants utilisables dans l'invention, on peut citer les alcools inférieurs, notamment l'éthanol et l'isopropanol, le propylène glycol.

Comme gélifiants hydrophiles utilisables dans l'invention, on peut citer les polymères carboxyvinyliques (carbomer), les copolymères acryliques tels que les copolymères d'acrylates/alkylacrylates, les polyacrylamides, les polysaccharides tels que l'hydroxypropylcellulose, les gommes naturelles et les argiles, et, comme gélifiants lipophiles, on peut citer les argiles modifiées comme les bentones, les sels métalliques d'acides gras comme les stéarates d'aluminium et la silice hydrophobe, éthylcellulose, polyéthylène.

La composition peut contenir d'autres actifs hydrophiles comme les protéines ou les hydrolysats de protéine, les acides aminés, les polyols, l'urée, l'allantoïne, les sucres et les dérivés de sucre, les vitamines hydrosolubles, les extraits végétaux et les hydroxyacides.

Comme actifs lipophiles, on peut utiliser le rétinol (vitamine A) et ses dérivés, le tocophérol (vitamine E) et ses dérivés, les acides gras essentiels, les céramides, les huiles essentielles, l'acide salicylique et ses dérivés.

Selon l'invention la composition peut associer au moins un composé de formule (I) à d'autres agents actifs. Parmi ces agents actifs, on peut citer à titre d'exemple :
- les agents améliorant la repousse et/ou sur le ralentissement de la chute des cheveux, et ayant déjà été décrits pour cette activité comme par exemple les esters d'acide nicotinique, dont notamment le nicotinate de tocophérol, le nicotinate de benzyle et les nicotinates d'alkyles en C₁-C₆ comme les nicotinates de méthyle ou d'hexyle, les dérivés de pyrimidine, comme le 2,4-diamino 6-piperidinopyrimidine 3-oxyde ou "Minoxidil" décrit dans les brevets US 4 139 619 et US 4 596 812, les agents favorisant la repousse des cheveux comme ceux décrits par la demanderesse dans la demande de brevet européen publiée sous le numéro 0648488 ;
- les agents modulant la différenciation et/ou la prolifération et/ou la pigmentation cutanée tels que l'acide rétinoïque et ses isomères, le rétinol et ses esters, la vitamine D et ses dérivés, les oestrogènes tels que l'oestradiol, l'acide kojique ou l'hydroquinone ;
- les antibactériens tels que le phosphate de clindamycine, l'érythromycine ou les antibiotiques de la classe des tétracyclines ;
- les antiparasitaires, en particulier le métronidazole, le crotamiton ou les pyréthrinoïdes ;
- les antifongiques, en particulier les composés appartenant à la classe des imidazoles tels que l'éconazole, le kétoconazole ou le miconazole ou leurs sels, les composés polyènes, tels que l'amphotéricine B, les composés de la famille des allylamines, tels que la terbinafine, ou encore l'octopirox ;
- les agents antiviraux tels que l'acyclovir ;
- les agents anti-inflammatoires stéroïdiens, tels que l'hydrocortisone, le valérate de bétaméthasone ou le propionate de clobétasol, ou les agents anti-inflammatoires non-stéroïdiens comme par exemple l'ibuprofène et ses sels, le diclofénac et ses sels, l'acide acétylsalicylique, l'acétaminophène ou l'acide glycyrrhizique ;
- les agents anesthésiques tels que le chlorhydrate de lidocaïne et ses dérivés ;
- les agents antiprurigineux comme la thénaldine, la triméprazine ou la cyproheptadine ;
- les agents kératolytiques tels que les acides α- et β-hydroxycarboxyliques ou β-cétocarboxyliques, leurs sels, amides ou esters et plus particulièrement les hydroxyacides tels que l'acide glycolique, l'acide lactique, l'acide salicylique, l'acide citrique et de manière générale les acides de fruits, et l'acide n-octanoyl-5-salicylique ;
- les agents anti-radicaux libres, tels que l'α-tocophérol ou ses esters, les superoxyde dismutases, certains chélatants de métaux ou l'acide ascorbique et ses esters ;
- les anti-séborrhéiques tels que la progestérone ;
- les antipelliculaires comme l'octopirox ou la pyrithione de zinc ;
- les antiacnéiques comme l'acide rétinoïque ou le peroxyde de benzoyle ;
- les extraits d'origine végétale, marine ou bactérienne.

A la liste ci-dessus, d'autres composés peuvent également être rajoutés, à savoir par exemple le Diazoxyde, la Spiroxazone, des phospholipides comme la lécithine, les acides linoléique et linolénique, l'acide salicylique et ses dérivés décrits dans le brevet français FR 2 581 542, comme les dérivés de l'acide salicylique porteurs d'un groupement alcanoyle ayant de 2 à 12 atomes de carbone en position 5 du cycle benzénique, des acides hydroxycarboxyliques ou cétocarboxyliques et leurs esters, des lactones et leurs sels correspondants, l'anthraline, des caroténoides, les acides eicosatétraénoïque et eicosatriénoïque ou leurs esters et amides, la vitamine D et ses dérivés, des extraits d'origine végétale ou bactérienne.

Ainsi, selon un mode particulier, la composition selon l'invention comprend également au moins un agent choisi parmi les agents antibactériens, les antiparasitaires, les antifongiques, les antiviraux, les anti-inflammatoires, les antiprurigineux, les anesthésiques, les kératolytiques, les anti-radicaux libres, les anti-séborrhéiques, les antipelliculaires, les antiacnéiques et/ou les agents diminuant la différenciation et/ou la prolifération et/ou la pigmentation cutanée, les extraits d'origine végétale, marine ou bactérienne.

La composition pharmaceutique selon l'invention peut être administrée par voie parentérale, entérale ou encore par voie topique. De préférence, la composition pharmaceutique est administrée par voie topique.

L'invention est illustrée plus en détail dans les exemples suivants.

Exemple 1 : On étudie, dans cet exemple, la capacité de dérivés de l'acide 10-hydroxy-2-décénoique à favoriser la desquamation.
Ce test de criblage *in vitro* d'un agent actif sur la desquamation est réalisé sur kératinocytes humains différenciés. Le principe du test repose sur le fait que la desquamation induit la libération de cornéocytes. Le pouvoir de desquamation du produit testé va être d'autant plus grand que le nombre de cornéocytes libéré sera important.

Le protocole du test a été le suivant : à partir de biopsies de peau humaine, les kératinocytes obtenus par séparation de l'épiderme sont dissociés par action enzymatique à la trypsine et mis en culture à la concentration de 2.10⁵ cellules/ml. La croissance et la différenciation des kératinocytes a été obtenue par culture durant 10 à 20 jours en milieu spécifique. Puis, après élimination du milieu de culture, l'activité du produit à tester est évalué. Pour ce faire, deux prélèvements à T0 et T60 ont été réalisés, c'est-à-dire avant l'ajout du produit et 60 minutes après cet ajout. Les prélèvements ainsi effectués ont été analysés au cytomètre de flux pour dénombrer la population de cornéocytes. Le cytomètre de flux permet de distinguer les populations de cornéocytes et de kératinocytes par traitement à l'acridine orange spécifique de l'ADN des cellules. Cette coloration est spécifique des kératinocytes puisque les cornéocytes normaux ne possèdent pas de noyaux donc par d'ADN.

L'indice de détachement cellulaire est déterminé par la différence entre T60 et T0. La même mesure a été réalisée pour un témoin ne contenant pas de produit à tester car l'expérience produit inévitablement la libération de cornéocytes, même en absence d'actifs.

Le test a été effectué avec des composés à la concentration de 5.10⁻⁵M.

Les composés suivants ont été testés :
1,2,3-tri-(10-hydroxy-dec-2-ènoate) de propyle,
et 1,2-di-(10-hydroxy-dec-2-ènoate) de 3-[(2-méthoxy)-éthoxy-méthoxy]-propyle,

Les résultats de cette étude sont résumés dans le tableau suivant :

| composés à 5 10⁻⁵ M | %** |
|---|---|
| Référence* | 91,95 |
| 1,2,3-tri-(10-hydroxy-dec-2-ènoate) de propyle | 148,52 |
| 1,2-di-(10-hydroxy-dec-2-ènoate) de 3-[(2-méthoxy)-éthoxy-méthoxy]-propyle | 124,16 |

| | |
|---|---|
| * Référence : Acide 2-hydroxy-5-octanoylbenzoïque connu comme favorisant la desquamation (Brevet FR-85-06953 de la demanderesse) | |
| ** : % d'activité par rapport au témoin constitué d'une culture identique en l'absence de composé. | |

Ces résultats montrent que l'activité des dérivés sur le détachement cellulaire est importante.

Exemple 2 : Exemples de formulations illustrant l'invention. Ces compositions ont été obtenues par simple mélange des différents composants.

### Composition 1 : Lait pour le visage

- Huile de vaseline 7,0 g
- 1,2-di-(10-hydroxy-dec-2-ènoate) de 3-[(2-méthoxy)-éthoxy-méthoxy]-propyle 1,0 g
- Monostéarate de glycéryle, stéarate de polyéthylène glycol (100 OE) 3,0 g
- Polymère carboxyvinylique 0,4 g
- Alcool stéarylique 0,7 g
- Protéines de soja 3,0 g
- NaOH 0,4 g
- Conservateur qs
- Eau qsp 100 g

Cette composition se présente sous forme d'un lait pour le visage, ayant de bonnes propriétés cosmétiques, et étant doux et confortable à utiliser.
Le pH de la composition est d'environ 5,5.

### Composition 2 : Lotion

- 1,3-di-(10-hydroxy-dec-2-ènoate) de 2-hydroxypropyle 0,5 g
- Palmitate d'éthyl-2 hexyle 10,0 g
- Cyclopentadiméthylsiloxane 20,0 g
- Butylène glycol 5,0 g
- Conservateur qs
- Eau qsp 100 g

Cette lotion, qui ne contient pas de tensioactif, favorise la desquamation de la peau.

### Composition 3 : Lait

- Palmitate d'octyle 35,0 g
- Glycérine 2,0 g
- 1,2,3-tri-(10-hydroxy-dec-2-ènoate) de propyle, 2,0 g
- Polymère réticulé acrylates C10-C30/alkylacrylates 0,1 g
- Triéthanolamine 0,1 g
- Acides aminés de blé 1,0 g
- Conservateur qs
- Eau qsp 100g

Le lait obtenu, qui ne contient pas de tensioactif, possède de bonnes propriétés cosmétiques.

### Composition 4 : Gel pour le visage

- Glycérine 10,0 g
- 1,2-di-(10-hydroxy-dec-2-ènoate) de 3-[(2-méthoxy)-éthoxy-méthoxy]-propyle 2, 0 g
- Cocoamphodiacétate de disodium 1,0 g
- Conservateur qs
- Eau qsp 100 g

Le gel obtenu possède de bonnes propriétés cosmétiques.

### Composition 5 : Gel nettoyant à l'eau

- Butylène glycol 7,0 g
- Sarcosinate de lauroyl sodium 4,0 g
- 1,2,3-tri-(10-hydroxy-dec-2-ènoate) de propyle 5,0 g
- Triéthanolamine 0,8 g
- Carbomer 0,5 g
- Conservateur qs
- Eau qsp 100 g

Le gel obtenu possède de bonnes propriétés cosmétiques.

## Revendications

1. Utilisation pour la préparation d'une composition, d'une quantité efficace d'au moins un dérivé de l'acide 10-hydoxy-2-décénoïque choisi parmi les esters suivants :
10-hydroxy-dec-2-ènoate de 2-disnéthylaminoéthyle de formule : 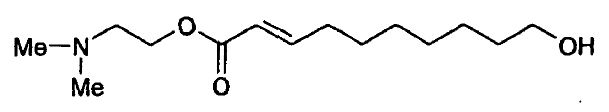
10-hydroxy-dec-ènoate de 2,3-dihydroxypropyle de formule : 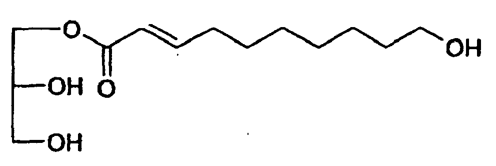
1,3-di-(10-hydroxy-dec-2-ènoate) de 2-hydroxypropyle de formule: 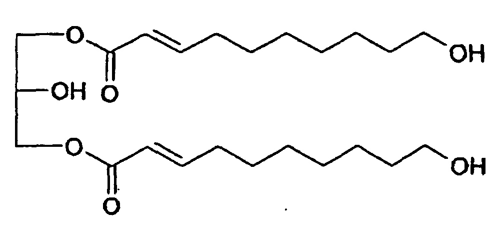
1,2,3-tri-(10-hydroxy-dec-2-ènoate) de propyle de formule : 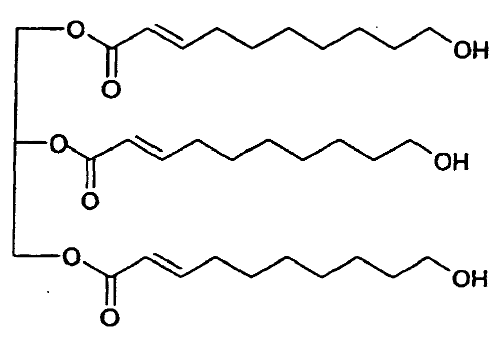
1,2-di-(10-hydroxy-dec-2-ènoate) de 3-[(2-méthoxy)-éthoxy-méthoxy]-propyle de formule : 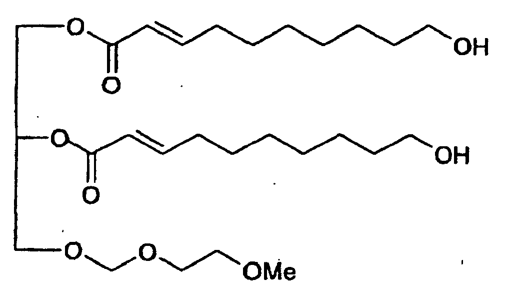
la composition étant destinée à favoriser la desquamation de la peau et/ou stimuler le renouvellement épidermique et donc lutter contre le vieillissement cutané intrinsèque et/ou extrinsèque.

2. Utilisation selon la revendication précédente, **caractérisée par le fait que** le dérivé de l'acide 10-hydroxy-2-décénoique est utilisé en une quantité allant de 0,001 % à 10 % du poids total de la composition.

3. Utilisation selon l'une quelconque des revendications précédentes, **caractérisée par le fait que** le dérivé de l'acide 10-hydroxy-2-décénoïque est utilisé en une quantité allant 0,01 % à 1 % du poids total de la composition.

4. Utilisation selon l'une quelconque des revendications précédentes, pour la préparation d'une composition se présentant sous la forme d'une émulsion, d'une solution aqueuse, éventuellement gélifiée, d'une lotion notamment biphasée, d'une crème, d'un lait, d'une mousse.

5. Composition pour favoriser la desquamation de la peau et/ou stimuler le renouvellement épidermique et donc lutter contre le vieillissement cutané intrinsèque et/ou extrinsèque, **caractérisée en ce qu'**elle comprend au moins un dérivé de l'acide 10-hydroxy-2-décénoïque tel que défini dans l'une des revendications 1 à 4.

6. Procédé de traitement non thérapeutique pour favoriser la desquamation de la peau et/ou stimuler le renouvellement épidermique et donc lutter contre le vieillissement cutané intrinsèque, contre le ralentissement du renouvellement des cellules de la peau, contre l'apparition de fines rides, de rides épaisses ou de ridules, contre la formation d'une peau molle et tanunée, contre le jaunissement de la peau, contre la perte d'élasticité, de souplesse et de fermeté de la peau, **caractérisé en ce qu'**on applique sur la peau une composition cosmétique telle que définie dans la revendication 5.

## Patentansprüche

1. Verwendung einer wirksamen Menge mindestens eines 10-Hydroxy-2-decensäure-Derivats, das aus den folgenden Estern ausgewählt ist:
2-Dimethylaminoethyl-10-hydroxydec-2-enoat der Formel: 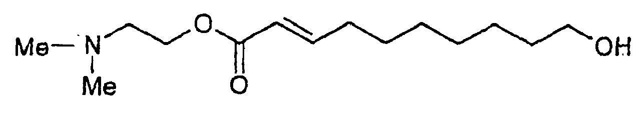
2,3-Dihydroxypropyl-10-hydroxydecenoat der Formel: 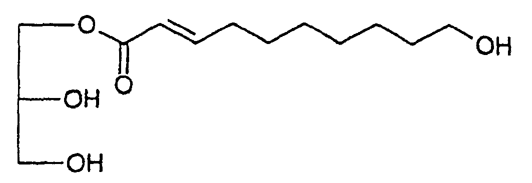
2-Hydroxypropyl-1,3-di-(10-hydroxydec-2-enoat) der Formel: 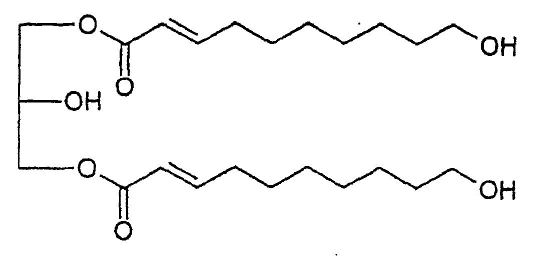
Propyl-1,2,3-tri-(10-hydroxydec-2-enoat) der Formel: 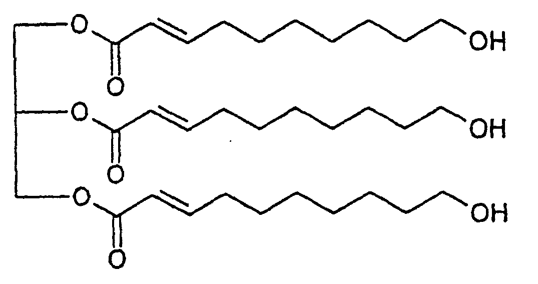
3-[(2-Methoxy)ethoxymethoxy]propyl-1,2-di-(10-hydroxydec-2-enoat) der Formel: 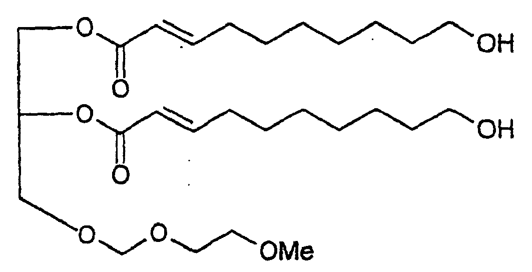
für die Herstellung einer Zusammensetzung, wobei die Zusammensetzung dazu bestimmt ist, die Abschuppung der Haut zu begünstigen und/oder die Epidermis-Emeuerung zu stimulieren und demgemäß die endogene und/oder exogene Hautalterung zu bekämpfen.

2. Verwendung nach dem vorangehenden Anspruch, **dadurch gekennzeichnet, dass** das 10-Hydroxy-2-decensäure-Derivat in einer Menge von 0,001 % bis 10 % des Gesamtgewichts der Zusammensetzung verwendet wird.

3. Verwendung nach irgendeinem der vorangehenden Ansprüche, **dadurch gekennzeichnet, dass** das 10-Hydroxy-2-decensäure-Derivat in einer Menge von 0,01% bis 1 % des Gesamtgewichts der Zusammensetzung verwendet wird.

4. Verwendung nach irgendeinem der vorangehenden Ansprüche für die Herstellung einer Zusammensetzung, die in Form einer Emulsion, einer gegebenenfalls gelatinierten wässrigen Lösung, einer insbesondere zweiphasigen Lotion, einer Creme, einer Milch, eines Schaums vorliegt.

5. Zusammensetzung, um die Abschuppung der Haut zu begünstigen und/oder die Epidermis-Erneuerung zu stimulieren und demgemäß die endogene und/oder exogene Hautalterung zu bekämpfen, **dadurch gekennzeichnet, dass** sie mindestens ein 10-Hydroxy-2-decensäure-Derivat, wie in einem der Ansprüche 1 bis 4 definiert, umfasst.

6. Verfahren zur nicht-therapeutischen Behandlung, um die Abschuppung der Haut zu begünstigen und/oder die Epidermis-Emeuerung zu stimulieren und demgemäß die endogene Hautalterung, die Verlangsamung der Erneuerung der Zeiten der Haut, das Erscheinen von feinen Falten, von starken Falten oder von Fältchen, die Bildung einer schlaffen und gegerbten Haut, die Gelbfärbung der Haut, den Verlust der Elastizität, der Geschmeidigkeit und der Festigkeit der Haut zu bekämpfen, **dadurch gekennzeichnet, dass** man auf die Haut eine kosmetische Zusammensetzung, wie in Anspruch 5 definiert, aufträgt.

## Claims

1. Use, for the preparation of a composition, of an effective amount of at least one 10-hydroxy-2-decenoic acid derivative chosen from the following esters:
2-dimethylaminoethyl 10-hydroxydec-2-enoate of formula: 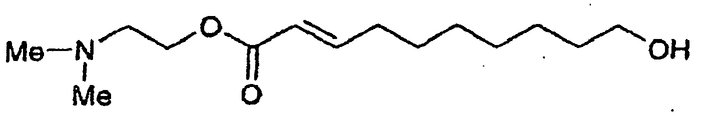
2,3-dihydroxypropyl 10-hydroxydec-2-enoate of formula: 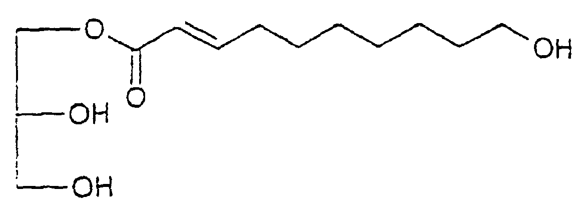
2-hydroxypropyl 1,3-di(10-hydroxydec-2-enoate) of formula: 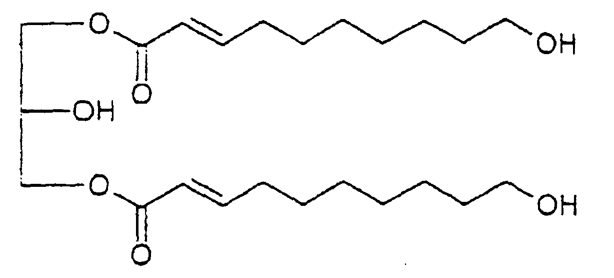
propyl 1,2,3-tri(10-hydroxydec-2-enoate) of formula: 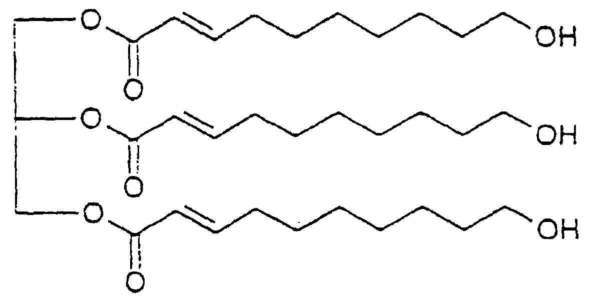
3-[(2-methoxyethoxy)methoxy]propyl 1,2-di(10-hydroxydec-2-enoate) of formula: 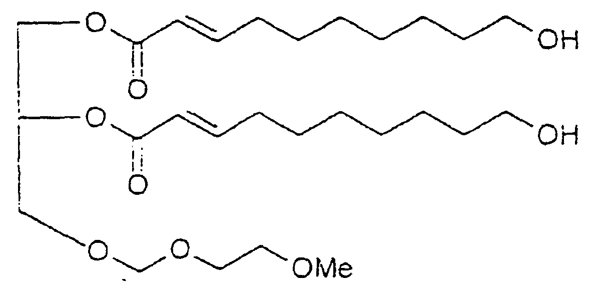
the composition being intended to promote desquamation of the skin and/or to stimulate epidermal renewal and thus to combat intrinsic and/or extrinsic cutaneous ageing.

2. Use according to the preceding claim, **characterized in that** the 10-hydroxy-2-decenoic acid derivative is used in an amount ranging from 0.001% to 10% of the total weight of the composition.

3. Use according to either one of the preceding claims, **characterized in that** the 10-hydroxy-2-decenoic acid derivative is used in an amount ranging from 0.01% to 1% of the total weight of the composition.

4. Use according to any one of the preceding claims, for the preparation of a composition which is provided in the form of an emulsion, of an aqueous solution, optionally a gel solution, of a lotion, in particular a two-phase lotion, of a cream, of a milk or of a foam.

5. Composition for promoting desquamation of the skin and/or stimulating epidermal renewal and thus combating intrinsic and/or extrinsic cutaneous ageing, **characterized in that** it comprises at least one 10-hydroxy-2-decenoic acid derivative as defined in one of Claims 1 to 4.

6. Nontherapeutic treatment process for promoting desquamation of the skin and/or stimulating epidermal renewal and thus combating intrinsic cutaneous ageing, the slowing down of the renewal of the cells of the skin, the appearance of small wrinkles, large wrinkles or fine lines, the formation of flabby and weathered skin, yellowing of the skin or loss of elasticity, suppleness and firmness of the skin, **characterized in that** a cosmetic composition as defined in Claim 5 is applied to the skin.
